# EUROPÄISCHE PATENTSCHRIFT

(11) **EP 3 125 013 B1**
(45) Veröffentlichungstag und Bekanntmachung des Hinweises auf die Patenterteilung: **14.02.2018**
(21) Anmeldenummer: 16165020.5
(22) Anmeldetag: 11.09.2014
(51) Int. Cl.: G02B 21/00, A61C 9/00, G01B 11/25, G02B 21/36, G02B 5/30, G02F 1/01

(54) **OPTISCHES SYSTEM ZUR ERZEUGUNG EINES SICH ZEITLICH ÄNDERNDEN MUSTERS FÜR EIN KONFOKALMIKROSKOP**
OPTICAL SYSTEM FOR THE GENERATION OF A PATTERN FOR A CONFOCAL MICROSCOPE WHICH CHANGES OVER TIME
SYSTEME OPTIQUE DESTINE A GENERER UN MODELE VARIANT DANS LE TEMPS POUR UN MICROSCOPE CONFOCAL

(30) Priorität: 11.09.2013 DE 102013218231
(43) Veröffentlichungstag der Anmeldung: 01.02.2017
(62) Teilanmeldung aus: 14783771.0
(73) Patentinhaber: Sirona Dental Systems GmbH, 64625 Bensheim (DE)
(72) Erfinder: Berner, Markus, 8180 Bülach (CH)
(74) Vertreter: Sommer, Peter

(56) Entgegenhaltungen:
- EP-A1- 2 051 042
- EP-A1- 2 136 233
- EP-A1- 2 258 254
- WO-A1-2011/139150
- WO-A2-2013/105922
- DE-A1-102007 018 048
- US-A- 3 897 136
- US-A1- 2011 141 483

## Beschreibung

### Technisches Gebiet

Die Erfindung betrifft ein optisches System zur Erzeugung eines sich zeitlich ändernden Musters für ein Konfokalmikroskop.

### Stand der Technik

Es gibt verschiedene Typen von Konfokalmikroskopen, welche eine Oberfläche eines zu scannenden Objekts aufgrund der Tatsache erkennen, dass sich die Oberfläche im Fokus befindet. Beispiele für solche Systeme sind Laser-Konfokalmikroskope, wie sie aus US2007/0109559 A1 bekannt sind, oder pOFPT (= parallel Focal Point Optical Tomography), wie es in der CH-Patentanmeldung 016247/07 beschrieben ist.

Bei einem pOFPT - Scanner wird ein bewegtes Muster auf ein zu scannendes Objekt projiziert. Dies ist deshalb vorteilhaft, weil es signaltechnisch einfacher und genauer ist, sich ändernde Muster zu detektieren als statische. Diese Bewegung wird dort so realisiert, dass eine Maske mit dem Muster mechanisch schnell bewegt wird. Die Bewegung erfolgt dabei oszillierend oder kontinuierlich.

Diese mechanische Bewegung hat aber einige erhebliche Nachteile. Die Mechanik benötigt Platz, was eine kompakte Bauweise unmöglich macht. Die Mechanik ist auch relativ teuer. Zudem ist die mechanische Bewegung nicht beliebig genau. Insbesondere ist die Genauigkeit der Geschwindigkeit der Änderung beschränkt, was den Aufwand für die Auswertung erheblich erhöht. Es kann bei der mechanischen Bewegung sogar zu Ausfällen im Betrieb führen.

Es gibt optische Elemente, die ein Bewegen bzw. Ändern des Musters erlauben würden. Diese Elemente werden beispielsweise bei Beamern angewendet. Jedoch sind sie für das gewünschte Scannen zu langsam. Zum Beispiel erlauben die LCDs für LCD-Beamer maximal ca. 200 Bildwechsel pro Sekunde. Für eine Anwendung beim Scannen mit dem hier beschriebenen System sind aber mindestens 2.000 Bildwechsel, besser jedoch 20.000 Bildwechsel, pro Sekunde nötig.

Es ist somit die Aufgabe der vorliegenden Erfindung, ein optisches System für ein Konfokalmikroskop und ein zugehöriges Verfahren zur Verfügung zu stellen, bei welchen insbesondere ein Bewegen bzw. Ändern des Musters vereinfacht ist und sich das Ändern zeitlich sehr genau kontrollieren und mit der nachfolgenden Auswertung der Signale exakt synchronisieren lässt.

### Darstellung der Erfindung

Diese Aufgabe wird durch ein optisches System gelöst, wie es im Anspruch 1 angegeben ist. Vorteilhafte Weiterbildungen der Erfindung sind in den abhängigen Ansprüchen angegeben. Erfindungsgemäss wird ein optisches System für ein Konfokalmikroskop mit einer Lichtquellenanordnung zur Verfügung gestellt, von welcher Lichtstrahlen zu einem Objekt gehen, das die Lichtstrahlen reflektiert, einem Strahlteiler zum Durchlassen der von der Lichtquellenanordnung ausgehenden Lichtstrahlen in Richtung zum Objekt und zum Ablenken der vom Objekt in einer Fokalebene reflektierten Lichtstrahlen in Richtung zu einem Detektor mit Detektormuster zum Detektieren eines Bilds des Objekts und einer Linsenanordnung zwischen dem Strahlteiler und dem Objekt. Die Lichtquellenanordnung weist wenigstens eine Lichtquelle und eine Einrichtung auf, die die von der wenigstens einen Lichtquelle ausgesendeten Lichtstrahlen bezüglich ihrer Polarisationsrichtung zum Generieren eines wechselnden Projektormusters ohne Bewegen einer Maske mit dem Projektormuster umschaltet.

Beim Strahlteiler handelt es sich vorzugsweise um einen polarisierenden Strahlteiler. Somit sollte das Licht des beweglichen Projektormusters stets die gleiche Polarisationsrichtung aufweisen.

Erfindungsgemäss wird somit die mechanische Bewegung durch eine starre, spezielle optische Anordnung und wenigstens eine polarisierte Lichtquelle, bei welcher die Polarisationsrichtung geändert wird, ersetzt. Das projizierte Muster bzw. Projektormuster ändert sich nun so schnell, wie die Polarisationsrichtung der Lichtquellenanordnung sich ändert. Die Änderung der Polarisationsrichtung kann beliebig schnell erfolgen. Erfindungsgemäß weist die Lichtquellenanordnung zwei Lichtquellen und eine Umschalteinrichtung als Einrichtung zum Umschalten der von der wenigstens einen Lichtquelle ausgesendeten Lichtstrahlen bezüglich ihrer Polarisationsrichtung auf, wobei dann die Lichtpfade der zwei Lichtquellen zu einem einzigen Lichtpfad kombiniert sind, wobei die zwei Lichtquellen polarisiertes Licht abgeben und die jeweiligen Polarisationsrichtungen der zwei Lichtquellen um 90° gedreht sind, und wobei die Umschalteinrichtung nur jeweils eine der Lichtquellen in einen eingeschalteten Zustand versetzt und somit die jeweilige Polarisationsrichtung des kombinierten Lichts wechselt.

Die zwei Lichtquellen können so dicht nebeneinander liegend vorgesehen sein, dass ihre Lichtpfade zu dem einzigen Lichtpfad kombiniert sind. Den Lichtquellen ist bevorzugt eine Streuscheibe nachgeschaltet. Eine solche Streuscheibe dient dazu, das auftreffende Licht besser zu bündeln und in die gewünschte Richtung abzustrahlen.

Die zwei Lichtquellen können auch um 90° versetzt angeordnet sein, wobei dann ein Strahlkombinierer ihre Lichtpfade zu einem einzigen Lichtpfad kombiniert.

Durch die voranstehend beschriebenen Ausführungsformen mit zwei Lichtquellen und einer Umschalteinrichtung ist ein besonders genaues Schalten zu einer jeweiligen Polarisationsrichtung möglich.

Die jeweiligen Lichtquellen sind bevorzugt Laserdioden, die schon von sich aus polarisiertes Licht abgeben, oder LEDs mit nachgeschalteten Polarisatoren. Solche Lichtquellen sind auf einfache Weise einzusetzen.

Das Licht ist bevorzugt linear polarisiert und die beiden Polarisationsrichtungen sind bevorzugt um 90° zueinander gedreht, so dass einfach strukturierte Polarisationsrichtungen verwendet werden. Erfindungsgemäß ist ein der Lichtquellenanordnung nachgeschalteter Lambda/4-Verzögerer vorgesehen, der die Polarisationsrichtungen in zwei zirkulare Polarisationsrichtungen umwandelt, von welchen eine linksdrehend und eine rechtsdrehend ist. Durch diese Umwandlung der Polarisationsrichtung kann ein Projektormuster auf einfache Weise generiert werden.

Zwischen einem dem Lambda/4-Verzögerer nachgeschalteten strukturierten Verzögerer und der Linsenanordnung ist ein Linear-Polarisator vorgesehen ist, der das Licht von Stellen, bei welchen die Polarisation um 90° gedreht ist, blockiert und das Licht von Stellen, bei welchen die Polarisationsrichtung vorliegt, durchlässt, so dass an diesen Stellen das Beleuchtungsmuster transparent ist. Auch durch diese Massnahme kann das Generieren eines Projektormusters vereinfacht werden und sie gewährleistet, dass das Licht, das zum nachfolgenden Strahlteiler geht, immer die gleiche Polarisationsrichtung aufweist.

Das Beleuchtungsmuster bzw. Projektormuster ist bevorzugt ein beliebig steuerbares Muster, ein starres Linien-Muster oder ein starres Schachbrett-Muster. Dies sind in der Praxis einfach zu handhabende Muster.

Steuerbare Muster lasser sich einfach realisieren, indem man ein transparentes LCD einsetzt, wie es in LCD-Beamern verwendet wird. Durch die Anlegung eines geeigneten Bilds wird ein strukturierter Verzögerer generiert.

Vorzugsweise sind Mittel zum Ausrichten von Projektormuster und Detektormuster vorgesehen. Das Ausrichten der Muster ist notwendig, damit ein gutes Bild beim Einsetzen des optischen Systems als Scanner erzielt werden kann.

Als Mittel zur Auswertung erhaltener Signale ist vorzugsweise ein Filter vorgesehen, dessen Schaltfrequenz mit der Umschaltfrequenz der Polarisationsrichtung der Lichtquelle synchronisiert ist. Bevorzugt ist das Filter als Lock-in-Verstärker realisiert. Das Synchronisieren dient zum Erreichen eines gut erkennbaren Signals beim Scannen.

### Kurzbeschreibung der Zeichnungen

Die angegebenen und weitere Merkmale und Einzelheiten der Erfindung werden einem Fachmann auf dem Gebiet aus der folgenden detaillierten Beschreibung und den beigefügten Zeichnungen klarer, die Merkmale der vorliegenden Erfindung anhand eines Beispiels darstellen und wobei
- Figur 1: einen allgemeinen Aufbau eines optischen Systems für ein Konfokalmikroskop gemäss der vorliegenden Erfindung darstellt,
- Figur 2: eine Lichtquellenanordnung mit schaltbarer Polarisationsrichtung mittels einer Pockelszelle darstellt,
- Figur 3: eine Lichtquellenanordnung mit schaltbarer Polarisationsrichtung mittels bewegter (ortsfest rotierender) Verzögerungsplatte darstellt,
- Figur 4: eine Lichtquellenanordnung mit schaltbarer Polarisationsrichtung mittels zweier Lichtquellen gemäss der vorliegenden Erfindung darstellt,
- Figur 5: eine weitere Lichtquellenanordnung mit schaltbarer Polarisationsrichtung mittels zweier Lichtquellen gemäss der vorliegenden Erfindung darstellt,
- Figur 6: eine Anordnung zur Generierung eines wechselnden Streifenmusters gemäss der vorliegenden Erfindung darstellt,
- Figur 7: einen strukturierten Verzögerer gemäss der vorliegenden Erfindung darstellt,
- Figur 8: eine Darstellung für eine Positionierung des sich ändernden Musters ist,
- Figur 9: ein Projektormuster zeigt,
- Figur 10: ein Detektormuster zeigt,
- Figur 11: ein Projektormuster und ein Detektormuster vom Messobjekt her gesehen bei korrekter Ausrichtung zeigt,
- Figur 12: ein Projektormuster und ein Detektormuster vom Messobjekt her gesehen bei fehlerhafter Ausrichtung zeigt
- Figur 13: ein Signal an einem Pixel beim Durchstimmen der Fokuslage zeigt,
- Figur 14: ein resultierendes Signal an einem Bildpunkt nach einer Schmalbandfilterung zeigt, und
- Figur 15: ein resultierendes Signal an einem Bildpunkt nach einer Schmalbandfilterung für ein Scannen natürlicher Zähne zeigt.

### Ausführungsbeispiel

Im Folgenden wird die vorliegende Erfindung anhand bevorzugter Ausführungsformen unter Bezugnahme auf die Figuren detailliert erklärt werden.

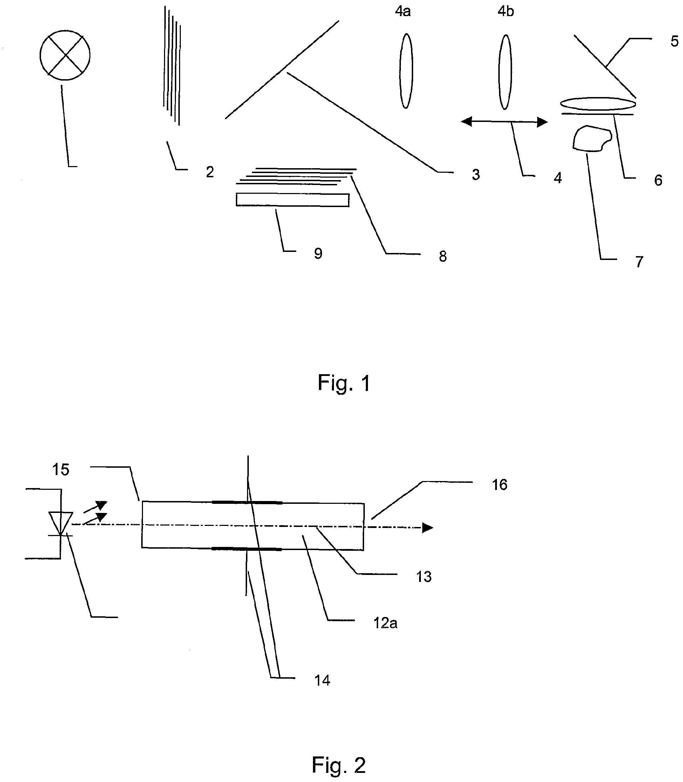

Figur 1 zeigt den allgemeinen Aufbau für ein erfindungsgemässes optisches System für ein Konfokalmikroskop, wobei eine Lichtquellenanordnung 1 vorgesehen ist und ein Projektormuster 2 ein projiziertes Muster ist, das sich je nach Polarisation der Lichtquellenanordnung 1 ändert. Die Lichtquellenanordnung 1 in Figur 1 ist in den Figuren 2 bis 4 detaillierter als Lichtquellenanordnung 1 mit schaltbarer Polarisation gezeigt. Weiterhin ist ein Strahlteiler 3 vorgesehen, der ein polarisierender Strahlteiler sein kann. Bei einem Doppelpfeil 4 erfolgt ein Verschieben der Fokalebene durch Bewegen der zweiten Linse 4b einer Linsenanordnung mit einer ersten Linse 4a und einer zweiten Linse 4b.

Es sind auch ein Umlenkspiegel 5, eine 1/4-Verzögerungsplatte 6, ein Objekt 7, wie beispielsweise ein Zahn, ein Detektormuster 8 und ein Sensor 9 gezeigt. Das Detektormuster muss nicht eingesetzt werden, wenn die Pixelstruktur des Sensors als Detektormuster verwendet wird.

Der Sensor kann beispielsweise ein Smart-Pixel-Bildsensor für pOCT sein.

Lichtstrahlen vom Projektormuster 2 laufen durch den Strahlteiler 3 in Richtung zum Objekt 7 durch die erste Linse 4a und die zweite Linse 4b bis zu einer Fokalebene beim Objekt 7. Die vom Objekt 7 reflektierten Lichtstrahlen laufen zurück durch die Linsen 4a und 4b und werden am Strahlteiler 3 in Richtung zum Sensor 9 abgelenkt, wo eine Abbildung des Objekts 7 detektiert wird. Bei einem Laserkonfokalmikroskop besteht das Beleuchtungsmuster bzw. Projektormuster aus mindestens einer Punktlichtquelle, dem Laser, und bei einem pOFPT-Gerät besteht das Beleuchtungsmuster aus einem Bild, welches von einer Lichtquelle durchstrahlt wird. Durch den Doppelpfeil 4 bei der zweiten Linse 4b ist eine Bewegung der zweiten Linse 4b angedeutet, welche Bewegung in einer entsprechenden Bewegung der Fokalebene am Objekt 7 resultiert. Zur Bewegung der zweiten Linse 4b ist eine Antriebsvorrichtung vorgesehen, die beispielsweise ein gesteuerter Motor sein kann.

Figur 2 zeigt eine Lichtquellenanordnung 1, 12a mit schaltbarer Polarisationsrichtung mittels Pockelszelle 12a mit elektrischen Anschlüssen 14, einen optischen Strahlengang 13, einen Lichteintritt 15 und einen Lichtaustritt 16.

Die Lichtquelle 1 gibt polarisiertes Licht ab. Sie kann zum Beispiel eine Laserdiode sein, die schon von sich aus polarisiertes Licht abgibt, oder auch eine LED mit einem nachgeschaltetem Polarisator. Die Pockelszelle 12a ist ein steuerbarer optischer Verzögerer, der die Polarisationsrichtung je nach angelegter Spannung an den elektrischen Anschlüssen 14 von Lichteintritt 15 nach Lichtaustritt 16 drehen kann.

Die Spannung wird vorzugsweise so zwischen zwei Spannungen V1 und V2 geschaltet, dass sich die Verzögerung um Lambda/2 oder ein ungerades Vielfaches davon ändert. So hat das Licht beim Lichtaustritt 16 bei den Spannungen V1 und V2 zwei unterschiedliche Polarisationsrichtungen, zum Beispiel zwei um vorzugsweise 90° gedrehte linear polarisierte Richtungen oder zwei zirkulare Polarisationsrichtungen, von denen eine linksdrehend und eine rechtsdrehend ist, oder entsprechende Mischungen.

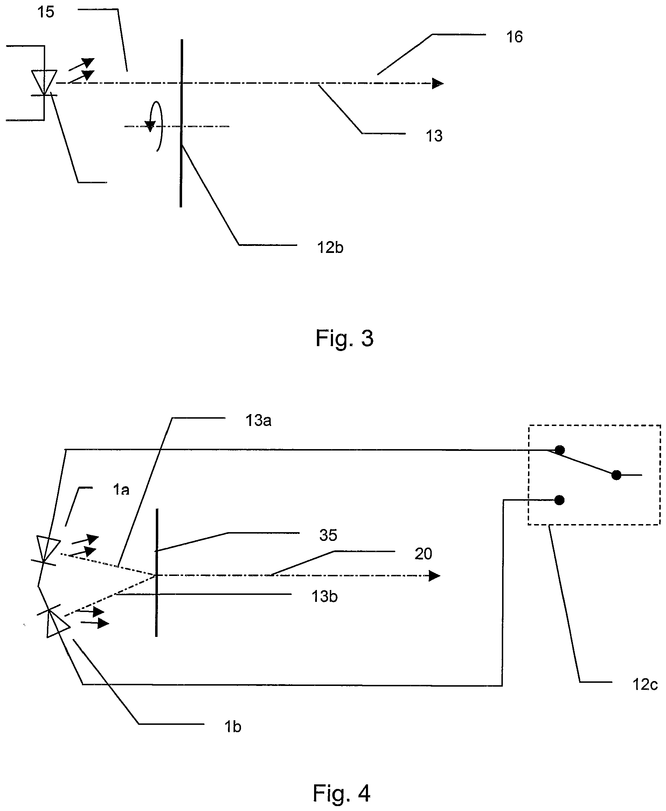

Die Figur 3 zeigt eine Lichtquellenanordnung mit einer Lichtquelle 1 und einem ortsfest rotierenden Lambda/4-Verzögerer 12b, einen optischen Strahlengang 13, einen Lichteintritt 15 und einen Lichtaustritt 16. Anstelle der Pockelszelle 12a der Figur 2 wird bei einem Aufbau gemäss der Figur 3 die Verzögerung und damit die Polarisation beim Lichtaustritt 16 mittels mechanischer Bewegung (hier Rotation) verändert.

Figur 4 zeigt eine Lichtquellenanordnung mit einer ersten Lichtquelle 1a und einer zweiten Lichtquelle 1b. Die zwei Lichtquellen 1a und 1b sind nebeneinander liegend angeordnet. Wenn sie sehr dicht nebeneinander liegend angeordnet sind, werden ein optischer Strahlengang 13a des Lichtpfads der ersten Lichtquelle 1a und ein optischer Strahlengang 13b des Lichtpfads der zweiten Lichtquelle 1b zu einem optischen Strahlengang 20 des Lichtpfads der kombinierten Lichtpfade der ersten Lichtquelle 1a und der zweiten Lichtquelle 1b.

Die Polarisationsrichtung des kombinierten Lichts kann nun mittels einer Umschalteinrichtung 12c geschaltet werden, indem man alternativ nur die Lichtquelle 1a und nur die Lichtquelle 1b einschaltet.

Die zwei Lichtquellen 1a und 1b geben polarisiertes Licht ab und die jeweiligen Polarisationsrichtungen der zwei Lichtquellen 1a und 1b sind um 90° gedreht. Die Umschalteinrichtung 12c versetzt nur jeweils eine der Lichtquellen 1a und 1b in einen eingeschalteten Zustand und wechselt somit die jeweilige Polarisationsrichtung des kombinierten Lichts.

Gemäß der Figur 4 ist den Lichtquellen 1a und 1b eine Streuscheibe nachgeschaltet, um das Licht besser zu bündeln und in die gewünschte Richtung abzustrahlen.

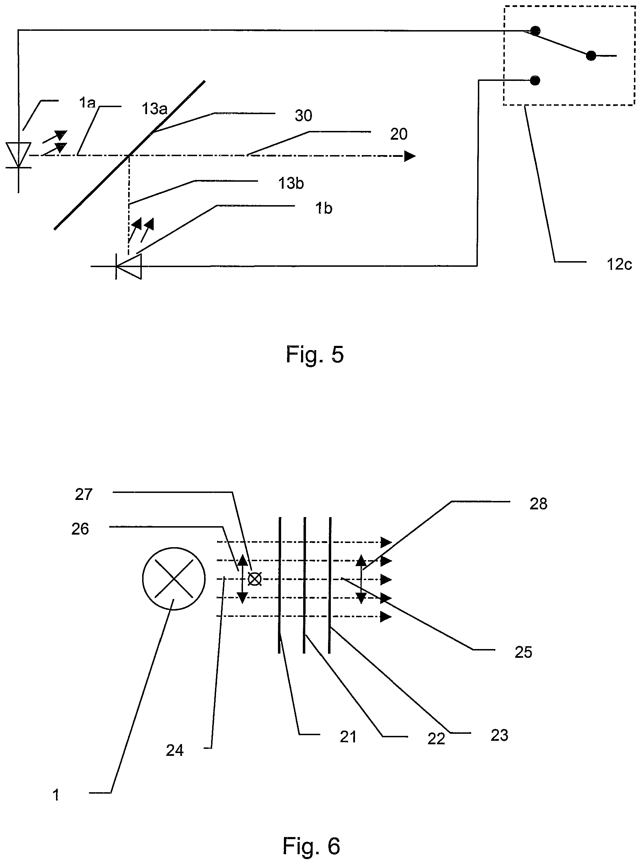

Auch die Figur 5 zeigt eine Lichtquellenanordnung mit einer ersten Lichtquelle 1a und einer zweiten Lichtquelle 1b. Die zwei Lichtquellen 1a und 1b sind um 90° versetzt angeordnet und ein polarisierender Strahlkombinierer/Strahlteiler 30 kombiniert ihre Lichtpfade zu einem einzigen Lichtpfad. In der Figur 5 ist weiterhin ein optischer Strahlengang 13a des Lichtpfads der ersten Lichtquelle 1a, ein optischer Strahlengang 13b des Lichtpfads der zweiten Lichtquelle 1b und ein optischer Strahlengang 20 des Lichtpfads der kombinierten Lichtpfade der ersten Lichtquelle 1a und der zweiten Lichtquelle 1b gezeigt.

Bei den Ausführungsformen der Figuren 4 und 5 geben die Lichtquellen 1a und 1b polarisiertes Licht ab. Sie können zum Beispiel jeweils eine Laserdiode sein, die schon von sich aus polarisiertes Licht abgibt, oder auch eine LED mit einem nachgeschaltetem Polarisator. Die beiden Lichtquellen 1a und 1b sind so konfiguriert, dass die Polarisationsrichtungen zwischen ihnen um 90° gedreht sind. Gemäß der Ausführungsform der Figur 5 kombiniert der Strahlkombinierer 30 die Lichtpfade 13a und 13b der beiden Lichtquellen 1a und 1b zu einem einzigen Lichtpfad 20. Gemäß der Ausführungsform der Figur 4 ergibt sich der einzige Lichtpfad 20 dadurch, dass die beiden Lichtquellen 1a und 1b dicht nebeneinander liegend angeordnet sind. Die Bündelung und Ausrichtung kann durch Vorsehen der dargestellten Streuscheibe verbessert werden.

Die Polarisationsrichtung des kombinierten Lichts kann nun mittels der Umschalteinrichtung 12c geschaltet werden, indem man alternativ nur die Lichtquelle 1a und nur die Lichtquelle 1b einschaltet.

Bei dieser Anordnung ist das Licht linear polarisiert und sind die beiden Polarisationsrichtungen um 90° zueinander gedreht. Bei Bedarf können durch Nachschalten eines Lambda/4-Verzögerers die Polarisationsrichtungen in zwei zirkulare Polarisationsrichtungen, von welchen eine linksdrehend und eine rechtsdrehend ist, umgewandelt werden.

Mittels eines strukturierten Verzögerers und eines nachgeschalteten Linearpolarisators kann man ein sich wechselndes Muster generieren - je nach Polarisationsrichtung der Beleuchtung wird das eine oder das andere Muster projiziert.

Die Figur 6 zeigt eine Anordnung zum Generieren eines wechselnden Streifenmusters, wobei insbesondere eine Lichtquelle 1, ein unstrukturierter Lambda/4-Verzögerer 21, ein strukturierter Verzögerer 22, ein Linear-Polarisator 23, Licht 24 von der Quelle, Licht 25 mit wechselndem Muster, eine erste Polarisationsrichtung 26 der Lichtquelle 1, eine zweite Polarisationsrichtung 27 der Lichtquelle und eine Polarisationsrichtung 28 des Lichts mit dem Muster gezeigt sind.

Die Lichtquelle 1 sendet Licht mit sich wechselnder Polarisationsrichtung 26, 27, wie beispielsweise linear polarisiert, aus. Der Lambda/4-Verzögerer 21 wandelt das linear polarisierte Licht in zirkular polarisiertes Licht um, wechselnd zwischen rechts- und linksdrehend, um. Der strukturierte Verzögerer 22 ist so strukturiert, dass er an bestimmten Stellen um +Lambda/4 und an anderen Stellen um - Lambda/4 verzögert.

Das Licht von der Lichtquelle 1 erfährt also an bestimmten Stellen eine Verzögerung um Lambda/2, nämlich +Lambda/4 vom Verzögerer 21 und +Lambda/4 vom strukturierten Verzögerer 22, und an anderen Stellen keine Verzögerung, nämlich +Lambda/4 vom Verzögerer 21 und -Lambda/4 vom strukturierten Verzögerer 22.

An den Stellen, an welchen das Licht eine Verzögerung um Lambda/2 erfährt, wird die Polarisationsrichtung um 90° gedreht. An den anderen Stellen wird sie nicht gedreht.

Ist nun die Lichtquelle 1 so geschaltet, dass die Polarisationsrichtung 26 vorliegt, so sind diejenigen Stellen des Musters transparent, an welchen keine Drehung der Polarisation erfolgt. Das Licht von den Stellen, an welchen die Polarisation um 90° gedreht wird, wird am Linear-Polarisator 23 geblockt. Ist umgekehrt die Lichtquelle 1 so geschaltet, dass die Polarisationsrichtung 27 vorliegt, so sind die anderen Stellen des Musters transparent.

Die Anordnung der Figur 6 ist eine mögliche und sinnvolle Anordnung zum Generieren eines geeigneten Projektormusters. Die Verzögerung kann aber auch auf andere Weise verteilt sein und der unstrukturierte Lambda/4-Verzögerer 21 ist nicht immer erforderlich und er kann auch zwischen den Elementen 22 und 23 angeordnet sein.

Wichtig ist jedoch, dass die Unterschiede der Verzögerungen am strukturierten Verzögerer 22 zumindest ungefähr Lambda/2 oder ein ungerades Vielfaches davon betragen, damit vor dem Linear-Polarisator 23 linear polarisiertes Licht mit um 90° verdrehter Polarisationsrichtung vorliegt.

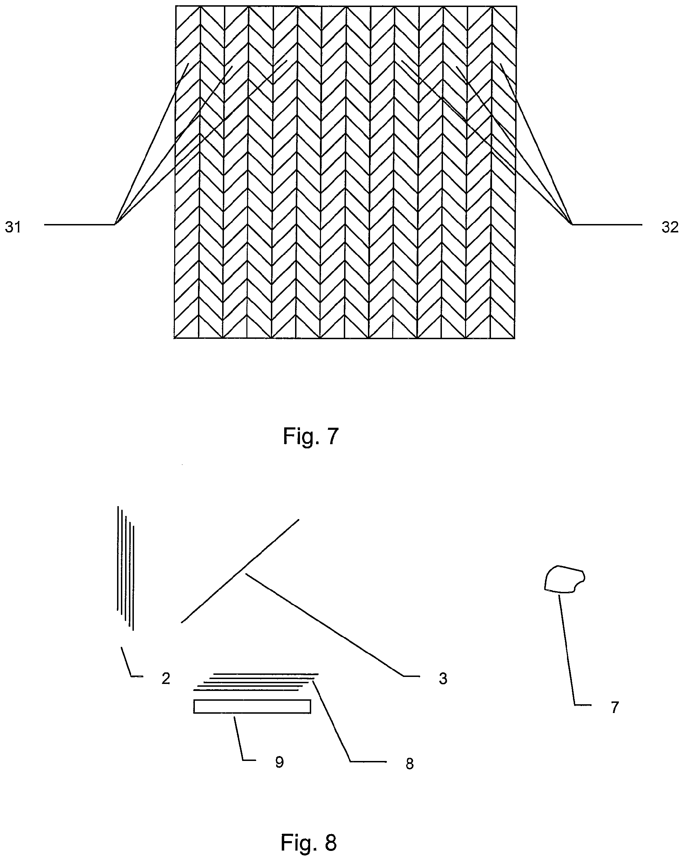

Um ein umschaltendes Streifenmuster als Projektormuster beispielsweise durch die in Figur 5 gezeigte Anordnung zu generieren, sieht der strukturierte Verzögerer zum Beispiel so aus, wie es in der Figur 7 gezeigt ist. In dieser Figur 7 sind Stellen mit +Lambda/4 Verzögerung 31 und Stellen mit -Lambda/4 Verzögerung 32 zu sehen.

Solche strukturierten Verzögerer sind heute Stand der Technik und werden zum Beispiel in 3D-Fernseher eingesetzt. Es ist auch möglich, einen transparenten LCD einzusetzen, wie er beispielsweise bei Beamern eingesetzt wird. Dies ist auch ein strukturierter Verzögerer, wobei die Zonen mit der jeweiligen Verzögerung wie das Bild auf dem LCD geschaltet werden können. Die strukturierten Verzögerer können jedes beliebige Muster aufweisen. Geeignete Muster für die erfindungsgemässe Anwendung sind zum Beispiel Linien- oder Schachbrettmuster.

Zum Erreichen eines guten Signals beim Einsatz des erfindungsgemässen optischen Systems müssen Projektormuster und Detektormuster des Systems ausgerichtet sein und entsprechend positioniert werden. Eine derartige Positionierung des sich ändernden Musters wird nun unter Bezugnahme auf die Figur 8 beschrieben. In der Figur 8 ist ein Projektormuster 2 als projiziertes Muster zu sehen, das sich je nach Polarisation der Lichtquelle ändert. Weiterhin sind ein Detektormuster 8, das bei Verwendung der Pixelstruktur des Sensors als Detektormuster nicht eingesetzt werden muss, ein Sensor 9, der ein Smart Pixel Bildsensor für pOCT sein kann, ein Strahlteiler 3, der ein polarisierender Strahlteiler sein kann, und ein Messobjekt 7 gezeigt.

Vom Messobjekt 7 her gesehen müssen das Projektormuster 2 und das Detektormuster 8 optisch am gleichen Ort angeordnet sein. Das Projektormuster 2 sieht das Objekt direkt und das Detektormuster 8 sieht das Objekt über den Strahlteiler 3. Beide Muster erscheinen aber am gleichen Ort.

Hierzu ist anzumerken, dass die Anordnung von Projektormuster 2 und Detektormuster 8 vertauscht sein kann. Im Folgenden wird jeweils von Detektormuster 8 gesprochen, auch wenn kein solches Bauelement eingesetzt werden muss. Idealerweise wird direkt die Pixelstruktur des Sensors als Detektormuster verwendet.

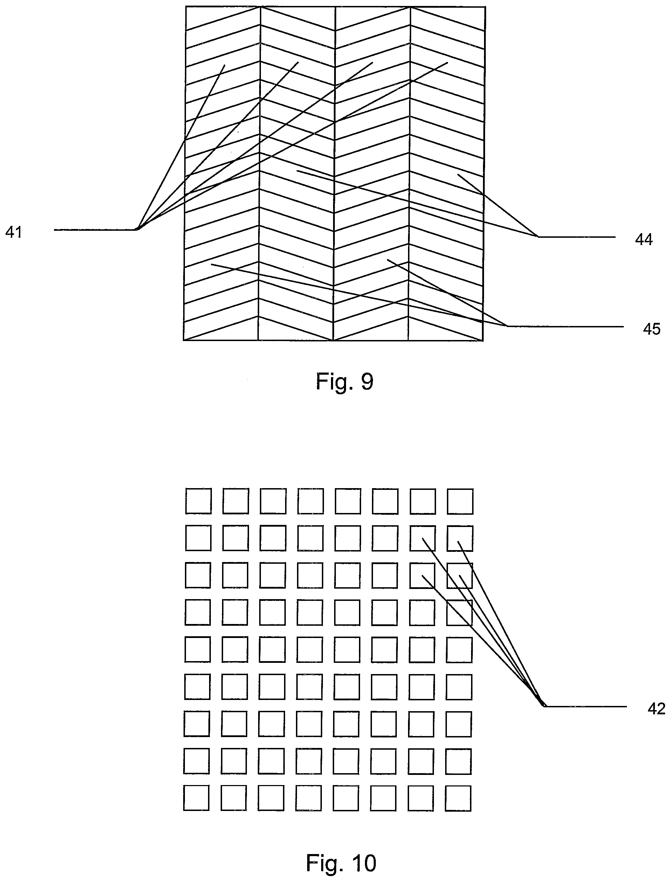

Es folgt nun eine Beschreibung einer lateralen Ausrichtung von Projektormuster und Detektormuster. Im folgenden Beispiel ist das Projektormuster ein Streifenmuster und ist das Detektormuster die Pixelstruktur eines Bildsensors, wie es in den Figuren 9 und 10 zu sehen ist. Dabei sind ein Projektormuster 41 ein Streifenmuster und ein Detektormuster 42 eine Pixelstruktur eines Bildsensors. Weiterhin sind eine erste Flächenart 44, die hell ist, wenn eine Polarisation der Quelle 1 in einem ersten Zustand ist, und dunkel ist, wenn die Polarisation in einem zweiten Zustand ist, und eine zweite Flächenart 45, die hell ist, wenn eine Polarisation der Quelle 1 im zweiten Zustand ist, und dunkel ist, wenn die Polarisation im ersten Zustand ist, gezeigt.

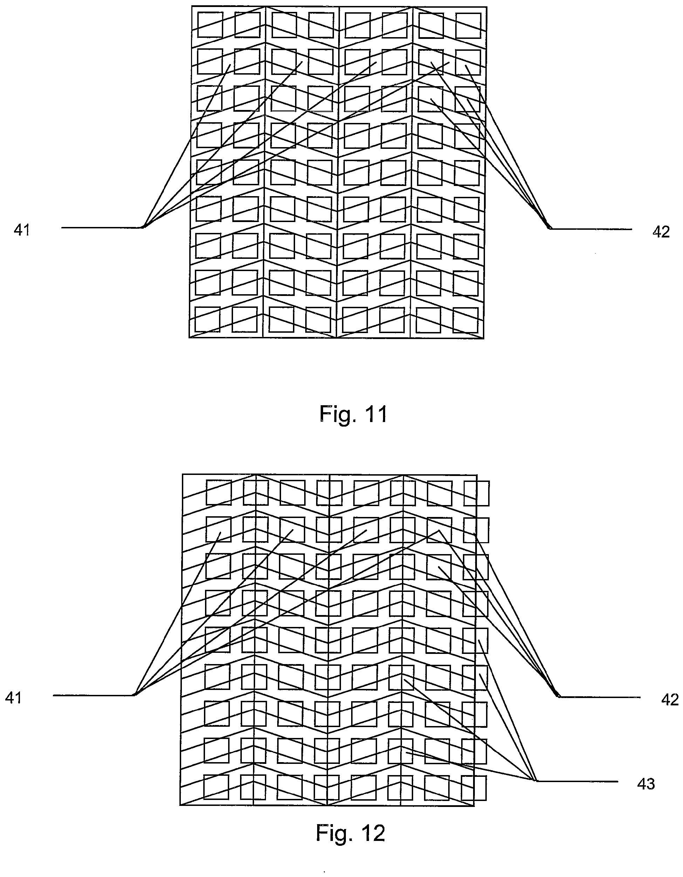

Die Figur 11 zeigt nun ein Projektormuster und ein Detektormuster vom Messobjekt her gesehen bei korrekter Ausrichtung und die Figur 12 zeigt ein Projektormuster und ein Detektormuster vom Messobjekt her gesehen bei fehlerhafter Ausrichtung.

In den Figuren 11 und 12 ist zusätzlich zu dem Projektormuster 41 als Streifenmuster, dem Detektormuster 42 als Pixelstruktur eines Bildsensors, der ersten Flächenart 44 und der zweite Flächenart 45 der Figuren 9 und 10 noch Pixel 43, welche kein Signal liefern werden, gezeigt.

Bei korrekter Ausrichtung, wie sie in der Figur 11 zu sehen ist, werden alle Detektor-Pixel ein Signal liefern. Bei fehlerhafter Ausrichtung, wie sie in der Figur 12 zu sehen ist, werden solche Detektor-Pixel 43, die in einem Übergang des Projektormusters liegen, kein oder nur ein schwaches Signal liefern.

Die laterale (seitliche) gegenseitige Ausrichtung kann in gewissen Fällen vereinfacht werden. Falls beim Projektormuster z.B. ein Display-LCD mit einer Pixelstruktur eingesetzt wird, welche feiner als die Struktur des Detektormusters ist, kann die Position des Musters mittels Software an die Lage des Detektormusters angepasst werden. Falls ein Detektormuster mit einer Struktur eingesetzt wird, die feiner als die erforderliche laterale Auflösung ist, wie z.B. ein hochauflösender Bildsensor, so kann auf eine Ausrichtung verzichtet werden, indem auf die Pixel, welche ein zu schwaches Signal liefern, verzichtet wird.

Es folgt eine Beschreibung der Auswertung des Signals. Bei der nachfolgenden Beschreibung wird davon ausgegangen, dass eine Lichtquelle 1a und eine Lichtquelle 1b gemäss Figur 5 verwendet werden. Mit entsprechenden Anpassungen gilt die Beschreibung aber auch für die anderen Anordnungen der Figuren 2 bis 4.

Das Scannen mit dem Scanner erfolgt nun wie folgt: Die Lage der Fokalebene wird durchgestimmt. Während des Durchstimmens wird laufend die Polarisation der Lichtquelle umgeschaltet, wie z.B. mit einer Frequenz f1. Ist das zu scannende Objekt weit genug von der Fokalebene des Projektormusters entfernt, sieht ein einzelnes Detektorpixel das Projektormuster so unscharf, dass von beiden Flächenarten 44 und 45 der Figur 8 gleich viel Licht auf das Pixel fällt. Die beiden Lichtquellen 1a und 1b der Beleuchtung werden so eingestellt, z.B. so bestromt, dass die gemessene Lichtmenge dann auch wirklich gleich ist.

Ist das zu scannende Objekt in der Fokalebene des Projektormusters, so treten auf jedem Pixel je nachdem, ob die Flächenarten 44 oder 45 gerade hell ist, starke Intensitätsunterschiede auf.

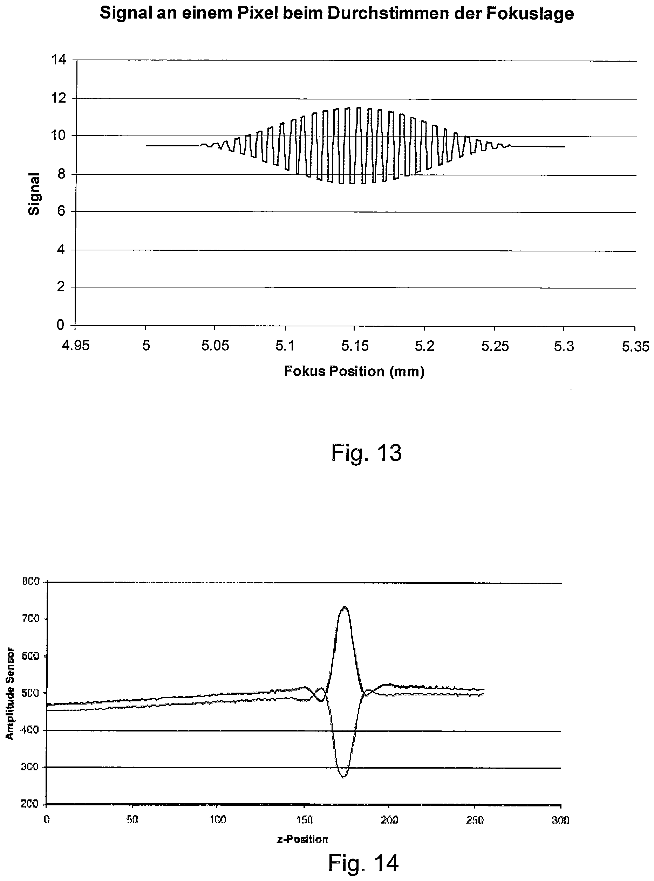

Das Signal sieht dann an einem Pixel etwa aus, wie es in der Figur 13 zu sehen ist.

Beim Beispiel der Figur 13 gibt es an der Stelle des entsprechenden Pixels bei 5.15 mm ein Objekt. Die Frequenz des Signals entspricht der Frequenz f1, mit welcher die Polarisation der Lichtquelle umgeschaltet wird.

Um die Stelle zu finden, wo sich das Objekt befindet, geht man wie folgt vor: Man führt eine Schmalbandfilterung des Signals mit der Frequenz f1 durch und sucht die maximale Intensität des Signals nach der Schmalbandfilterung.

Vorzugsweise wählt man die Schaltfrequenz relativ hoch, so dass bei der Oberfläche viele Perioden des Wechselsignals auftreten. Das erlaubt eine sehr schmalbandige Filterung des Wechselsignals, womit auch Rauschen und Brumm des Signals herausgefiltert werden.

Die Filterung wird idealerweise als digitales Filter realisiert, wobei die Schaltfrequenz des Filters exakt synchronisiert wird mit der Umschaltung der Polarisationsrichtung der Quelle. Dazu wird idealerweise ein Lock-in-Verstärker eingesetzt.

Durch bewusste Asymmetrien bei der Filterungen können so auch bei Bedarf Asymmetrien in der Beleuchtung kompensiert werden: Ist das Signal bei den beiden Polarisationsrichtungen nicht genau gleich stark, so ergibt sich auch entfernt vom Fokus ein gewisses Wechselsignal. Dies kann korrigiert werden, indem die Lichtstärken aneinander angeglichen werden oder auch indem gezielt asymmetrisch gefiltert wird.

Diese Schmalbandfilterung erfolgt idealerweise mittels Smartpixel Sensor für OCT-(OCT = Optische Kohärenztomografie) Anwendungen. Dort wird diese Filterung bei jedem Pixel auf dem Sensor durchgeführt.

Das resultierende Signal an einem Bildpunkt sieht nach obiger Schmalbandfilterung nun aus, wie es in der Figur 14 zu sehen ist.

Die z-Position ist ein Mass für die Position der Fokusebene. An der Stelle des lokalen Maximums bzw. Minimums befindet sich beim entsprechenden Bildpunkt die Oberfläche.

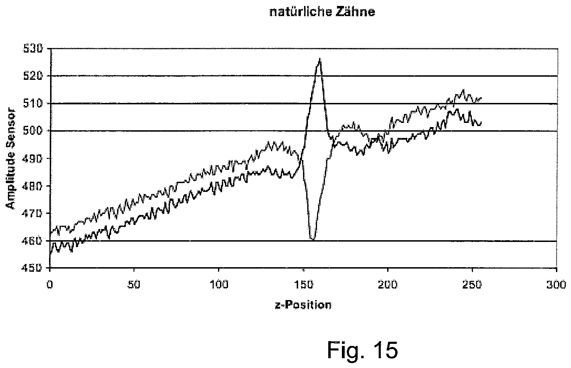

Weniger kooperative Oberflächen, wie zum Beispiel das Material natürlicher Zähne, ergeben ein ähnliches, wenn auch schwächeres Signal, wie es in der Figur 15 zu sehen ist.

Im Idealfall wäre das Signal über die Z-Position konstant und hätte nur an der Stelle, an welcher das Muster scharf auf das Objekt projiziert wird, ein Maximum oder ein Minimum. In der Praxis ist das Signal in keinem Fokusbereich wirklich konstant. Die Gründe dafür sind ein dem Signal überlagertes Rauschen und eine Drift des Signals aufgrund dessen, dass die beiden Lichtquellen mit der jeweils unterschiedlichen Polarisation nicht perfekt gleich sind.

Die Breite des Signalpeaks hängt hingegen vom optischen System des Messgeräts ab und ist unabhängig von der Art der Oberfläche des Messobjekts. Somit kann die Position der Oberfläche gefunden werden, indem ein Signalpeak bekannter Breite gesucht wird. Die Oberfläche wird also vorzugsweise so berechnet, indem nach einem Signalpeak dieser Breite gesucht wird. An der Stelle der maximalen oder minimalen Intensität eines solchen Peaks befindet sich dann die Oberfläche.

Erfindungsgemäss wird ein optisches System zur Erzeugung eines sich zeitlich ändernden Musters für ein Konfokalmikroskop zur Verfügung gestellt, bei welchem ein Bewegen des Projektormusters vereinfacht ist.

## Patentansprüche

1. Optisches System zur Erzeugung eines sich zeitlich ändernden Musters für ein Konfokalmikroskop, das optische System aufweisend eine Lichtquellenanordnung (1, 12a, 12b, 12c), von welcher Lichtstrahlen zu einem Objekt (7) gehen, das die Lichtstrahlen reflektiert, einen Strahlteiler (3) zum Durchlassen der von der Lichtquellenanordnung (1, 12a, 12b, 12c) ausgehenden Lichtstrahlen in Richtung zum Objekt (7) und zum Ablenken der vom Objekt (7) in einer Fokalebene reflektierten Lichtstrahlen in Richtung zu einem Detektor (8) mit Detektormuster zum Detektieren eines Bilds des Objekts (7), einem strukturierten Verzögerer (22) zum Erzeugen eines wechselnden Projektormusters (2) und eine Linsenanordnung (4a, 4b) zwischen dem Strahlteiler (3) und dem Objekt (7), **wobei** die Lichtquellenanordnung (1, 12a, 12b, 12c) wenigstens eine Lichtquelle (1) und eine Einrichtung (12a, 12b, 12c) aufweist, die die von der wenigstens einen Lichtquelle (1) ausgesendeten Lichtstrahlen bezüglich ihrer Polarisationsrichtung zum Generieren des wechselnden Projektormusters (2) ohne Bewegen einer Maske mit dem Projektormuster (2) umschaltet, wobei die Lichtquellenanordnung (1) zwei Lichtquellen (1a, 1b) und eine Umschalteinrichtung (12c) als Einrichtung zum Umschalten der Polarisationsrichtung der Lichtquellen (1a, 1b) aufweist, wobei die Lichtpfade (13a, 13b) der zwei Lichtquellen (1a, 1b) zu einem einzigen Lichtpfad (20) kombiniert sind, wobei die zwei Lichtquellen (1a, 1b) polarisiertes Licht abgeben und die jeweiligen Polarisationsrichtungen der zwei Lichtquellen (1a, 1b) um 90° gedreht sind, und wobei die Umschalteinrichtung (12c) nur jeweils eine der Lichtquellen (1a oder 1b) in einen eingeschalteten Zustand versetzt und somit jeweils die Polarisationsrichtung des kombinierten Lichts (20) wechselt, ein der Lichtquellenanordnung (1, 12a, 12b, 12c) nachgeschalteter Lambda/4-Verzögerer (21) vorhanden ist, der die Polarisationsrichtungen in zwei zirkulare Polarisationsrichtungen umwandelt, von welchen eine linksdrehend und eine rechtsdrehend ist, der strukturierte Verzögerer (22) dem Lambda/4-Verzögerer (21) nachgeschaltet ist und zwischen dem strukturierten verzögerer (22) und der Linsenanordnung (4a, 4b) ein Linear-Polarisator (23) vorhanden ist, der das Licht von Stellen, bei welchen die Polarisation um 90° gedreht ist, blockiert und das Licht von Stellen, bei welchen die Polarisationsrichtung vorliegt, durchlässt, so dass an diesen Stellen das Projektormuster transparent ist.

2. Optisches System nach Anspruch 1, **dadurch gekennzeichnet, dass** die zwei Lichtquellen (1a, 1b) so dicht nebeneinander liegend vorgesehen sind, dass ihre jeweiligen Lichtpfade (13a, 13b) zu dem einzigen Lichtpfad (20) kombiniert sind.

3. Optisches System nach Anspruch 2, **dadurch gekennzeichnet, dass** den Lichtquellen (1a, 1b) eine Streuscheibe (35) nachgeschaltet ist.

4. Optisches System nach Anspruch 2, **dadurch gekennzeichnet, dass** die zwei Lichtquellen (1a, 1b) um 90° versetzt angeordnet sind, wobei ein Strahlkombinierer (30) ihre Lichtpfade (13a, 13b) zu dem einzigen Lichtpfad (20) kombiniert.

5. Optisches System nach einem der vorangehenden Ansprüche, **dadurch gekennzeichnet, dass** die jeweilige Lichtquelle (1, 1a, 1b) eine Laserdiode ist, die schon von sich aus polarisiertes Licht abgibt, oder eine LED mit nachgeschaltetem Polarisator ist.

## Claims

1. An optical system for generating a pattern which changes over time for a confocal microscope comprising a light source arrangement (1, 12a, 12b, 12c) from which light beams travel to an object (7) which reflects the light beams, a beam splitter (3) for allowing passage of the light beams proceeding from the light source arrangement (1, 12a, 12b, 12c) in the direction toward the object (7) and for deflecting the light beams reflected by the object (7) in a focal plane in the direction toward a detector (8) comprising a detector pattern for detecting an image of the object (7), a structured retarder (22) for generating a changing projector pattern (2), and a lens arrangement (4a, 4b) between the beam splitter (3) and the object (7), wherein the light source arrangement (1, 12a, 12b, 12c) has at least one light source (1) and an arrangement (12a, 12b, 12c), which switches over the light beams emitted from the at least one light source (1) with respect to their direction of polarization to generate the changing projector pattern (2) without moving a mask with the projector pattern (2), wherein the light source arrangement (1) has two light sources (1a, 1b) and a switch-over device (12c) as the arrangement for switching over the direction of polarization of the light sources (1a, 1b), wherein the light paths (13a, 13b) of the two light sources (1a, 1b) are combined into a single light path (20), wherein the two light sources (1a, 1b) emit polarized light and the respective directions of polarization of the two light sources (1a, 1b) are rotated by 90°, and wherein the switch-over device (12c) shifts only one of the light sources (1a or 1b) at a time into a switched-on state, and thus respectively alternates the direction of polarization of the combined light (20), a lamda/4-retarder (21) is provided downstream of the light source arrangement (1, 12a, 12b, 12c) and converts the directions of polarization into two circular directions of polarization, of which one is clockwise and the other is counter-clockwise, the structured retarder (22) is arranged downstream of the lambda/4-retarder (21), and a linear polarizer (23) is provided between the structured retarder (22) and the lens arrangement (4a, 4b) and blocks the light from points at which the polarization is rotated by 90°, and allows the light to pass through from points at which the direction of polarization is present, so that the projector pattern is transparent at these points.

2. The optical system according to Claim 1, **characterized in that** the two light sources (1a, 1b) are provided so close to one another that their respective light paths (13a, 13b) are combined into the single light path (20).

3. The optical system according to Claim 2, **characterized in that** a diffusion disk (35) is downstream of the light sources (1a, 1b).

4. The optical system according to Claim 2, **characterized in that** the two light sources (1a, 1b) are arranged offset by 90°, wherein a beam combiner (30) combines their light paths (13a, 13b) into the single light path (20).

5. The optical system according to one of the preceding claims, **characterized in that** the respective light source (1, 1a, 1b) is a laser diode that emits already naturally polarized light, or is an LED with a downstream polarizer.

## Revendications

1. Système optique pour la production d'un motif variant dans le temps pour un microscope confocal, le système optique présentant un ensemble source de lumière (1, 12a, 12b, 12c) émettant des faisceaux lumineux dirigés vers un objet (7) qui réfléchissent les faisceaux lumineux, un séparateur de faisceaux (3) destiné à laisser passer les faisceaux lumineux provenant de l'ensemble source de lumière (1, 12a, 12b, 12c) en direction de l'objet (7) et à dévier les faisceaux lumineux, réfléchis par l'objet (7) dans un plan focal, en direction d'un détecteur (8) comportant un motif de détecteur visant à détecter une image de l'objet (7), un retardateur structuré (22) destiné à produire un motif de projecteur (2) changeant, et un agencement de lentilles (4a, 4b) entre le séparateur de faisceaux (3) et l'objet (7) ; dans lequel l'ensemble source de lumière (1, 12a, 12b, 12c) présente au moins une source de lumière (1) et un
dispositif (12a, 12b, 12c) qui change la direction de polarisation des faisceaux lumineux émis par l'au moins une source de lumière (1) afin de produire le motif de projecteur (2) changeant, sans déplacement d'un masque avec le motif de projecteur (2), ledit ensemble source de lumière (1) présentant deux sources de lumière (1a, 1b) et un dispositif de commutation (12c) servant de dispositif permettant de changer la direction de polarisation des sources de lumière (1a, 1b), les trajets lumineux (13a, 13b) des deux sources de lumière (1a, 1b) étant combinés en un trajet lumineux (20) unique, lesdites deux sources de lumière (1a, 1b) émettant une lumière polarisée et les directions de polarisation respectives des deux sources de lumière (1a, 1b) étant pivotées de 90°, le dispositif de commutation (12c) ne faisant passer qu'une des sources de lumière (1a ou 1b) respectives à un état allumé et changeant ainsi la direction de polarisation de ladite lumière combinée (20), un retardateur lambda/4 (21) étant présent en aval de l'ensemble source de lumière (1, 12a, 12b, 12c), lequel transforme les directions de polarisation en deux directions de polarisation circulaires, dont l'une a un sens de rotation vers la gauche et l'autre un sens de rotation vers la droite, le retardateur structuré (22) étant présent en aval du retardateur lambda/4 (21), et un polarisateur linéaire (23) étant présent entre le retardateur structuré (22) et l'agencement de lentilles (4a, 4b) pour bloquer la lumière provenant d'emplacements au niveau desquels la polarisation est pivotée de 90° et pour laisser passer la lumière provenant d'emplacements correspondant à la direction de polarisation, de sorte à rendre transparent le motif de projecteur au niveau de ces emplacements.

2. Système optique selon la revendication 1, **caractérisé en ce que** les deux sources de lumière (1a, 1b) sont prévues si proches l'une de l'autre que leurs trajets lumineux (13a, 13b) respectifs sont combinés en ledit trajet lumineux unique (20).

3. Système optique selon la revendication 2, **caractérisé en ce qu'**un diffuseur (35) est placé en aval des sources de lumière (1a, 1b).

4. Système optique selon la revendication 2, **caractérisé en ce que** les deux sources de lumière (1a, 1b) sont disposées de façon à être décalées de 90°, un combineur de faisceaux (30) combinant leurs trajets lumineux (13a, 13b) pour former ledit trajet lumineux unique (20).

5. Système optique selon l'une des revendications précédentes, **caractérisé en ce que** la source de lumière (1, 1a, 1b) respective est une diode laser, qui émet déjà par elle-même de la lumière polarisée ou une DEL pourvue d'un polarisateur en aval.
